(19) Europäisches Patentamt / European Patent Office / Office européen des brevets

(11) EP 2 238 896 B1

(12) FASCICULE DE BREVET EUROPEEN

(45) Date de publication et mention de la délivrance du brevet:
**22.06.2011 Bulletin 2011/25**

(51) Int Cl.:
*A61B 5/0402* *(2006.01)*   *A61B 5/042* *(2006.01)*
*A61B 5/04* *(2006.01)*   *A61N 1/37* *(2006.01)*

(21) Numéro de dépôt: **10156993.7**

(22) Date de dépôt: **19.03.2010**

(54) **Dispositif médical actif comprenant des moyens de reconstruction d'un électrocardiogramme de surface à partir de signaux de farfield extraits d'un électrogramme endocavitaire**

Aktive medizinische Vorrichtung, die Mittel zur Rekonstruktion eines Oberflächenelektrokardiogramms auf der Grundlage von Fernfeldsignalen aus einer endokavitären Aktionsstromkurve umfasst

Active medical device including means for reconstructing a surface electrocardiogram using far-field signals taken from an intracardiac electrogram

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **06.04.2009 FR 0901661**

(43) Date de publication de la demande:
**13.10.2010 Bulletin 2010/41**

(73) Titulaire: **Sorin CRM SAS**
**92541 Montrouge (FR)**

(72) Inventeur: **Makdissi, M. Alaa**
**75011, PARIS (FR)**

(74) Mandataire: **Dupuis-Latour, Dominique**
**SEP Bardehle Pagenberg Dost Altenburg Geissler**
**10, boulevard Haussmann**
**75009 Paris (FR)**

(56) Documents cités:
**EP-A- 1 897 587**   **EP-A- 1 902 750**
**US-B1- 7 349 732**

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme.

L'invention concerne plus particulièrement une technique de traitement des signaux représentatifs des potentiels cardiaques de dépolarisation du myocarde, signaux recueillis par des électrodes épicardiques ou endocavitaires de stimulation ou de détection ou de défibrillation des oreillettes ou des ventricules droits et gauches de ces dispositifs implantés.

Plus précisément, l'invention propose une technique, mise en oeuvre ou non dans ce dispositif implanté, permettant de reconstruire un électrocardiogramme de surface (ECG) à partir d'un électrogramme endocavitaire ou épicardique (EGM).

Les signaux EGM sont des signaux recueillis par des électrodes placées sur les sondes endocavitaires ou épicardiques implantées avec le dispositif. Ces signaux sont directement issus de l'activité électrique des cellules du myocarde et fournissent une information très riche pour évaluer l'état du patient. Après amplification, numérisation et filtrage, ils sont utilisés pour piloter le stimulateur cardiaque et pour diagnostiquer certains troubles du rythme nécessitant le déclenchement automatique d'une thérapie antitachycardique, antibradycardique ou de resynchronisation interventriculaire.

En revanche, lorsqu'il s'agit d'analyser de façon subjective le rythme pour établir un diagnostic ou pour réajuster les paramètres d'un implant, les médecins préfèrent en pratique interpréter les informations d'un électrocardiogramme de surface (ECG), qui permettent de visualiser de façon directe un certain nombre de facteurs importants (largeur du QRS, ...) et apprécier l'évolution d'une insuffisance cardiaque.

Ce sont également les signaux ECG qui sont enregistrés sur une longue période en ambulatoire par les dispositifs Holter, pour être ensuite traités et analysés afin d'évaluer l'état clinique du patient et diagnostiquer le cas échéant un trouble du rythme cardiaque.

Or les signaux ECG et EGM, bien qu'ils aient la même source (l'activité électrique du myocarde), se présentent visuellement de façon assez différente : l'EGM recueilli par la prothèse cardiaque fournit une information locale sur l'activité électrique d'un groupe de cellules cardiaques, tandis que l'ECG apparaît sous une forme plus globale, influencée en particulier par la propagation du signal électrique entre le myocarde et la surface du corps, et par un certain nombre de facteurs morphologiques et pathologiques. De ce fait, pour un médecin habitué à interpréter de longue date des ECG de surface, les signaux EGM ne sont généralement pas parlants.

Par conséquent, lorsqu'un patient porteur d'un implant vient voir son médecin pour une visite de contrôle, ce dernier utilise en pratique deux appareils, à savoir un enregistreur ECG et un programmateur d'implant. Pour recueillir l'ECG, le praticien colle un certain nombre d'électrodes sur le torse du patient, de manière à définir les douze "dérivations" usuelles correspondant à autant de signaux ECG différents. Le programmateur est, quant à lui, utilisé pour contrôler certains paramètres de fonctionnement de l'implant (par exemple la durée de vie de la pile), télécharger des données enregistrées dans la mémoire de l'implant, modifier éventuellement le paramétrage de ce dernier, y télécharger une version modifiée du logiciel de commande, etc.

La consultation chez le médecin requiert donc généralement deux appareils différents, ainsi qu'une manipulation pour la pose des électrodes de surface et le recueil des signaux ECG.

Le recours à ces deux appareils implique de plus le déplacement du patient dans un centre spécialement équipé (disposant du programmateur spécifique adapté à l'implant qu'il porte), avec pour conséquence le plus souvent un espacement des visites de contrôle et donc un moindre suivi du patient.

Par ailleurs, l'enregistrement d'un ECG présente divers inconvénients, notamment :

- la préparation du patient qui nécessite un certain temps, avec pour corolaire une augmentation du coût global du suivi ;
- l'irritation locale que peut créer la fixation des électrodes sur la peau de certains patients ;
- la position des électrodes qui varie d'une visite à l'autre, induisant des variations dans l'ECG recueilli,
- le fait que l'ECG est affecté par plusieurs paramètres qu'il est difficile de maîtriser, comme la respiration, les mouvements du patient, ainsi que les interférences provenant de sources électriques externes.

Pour remédier à ces multiples inconvénients, on a cherché à développer des algorithmes de reconstruction de l'ECG de surface à partir des signaux EGM, c'est-à-dire à partir des signaux directement délivrés par la prothèse implantée.

Une reconstruction de l'ECG de surface à partir des signaux EGM permettrait en effet :

- d'éviter, lors de la consultation, d'avoir à poser des électrodes et à recourir à un enregistreur ECG, avec tous les inconvénients et les imprécisions liés à cette technique ;
- de rendre ainsi la consultation plus simple et plus rapide, éventuellement de pouvoir la faire à domicile et par voie de conséquence rapprocher les contrôles et améliorer le suivi du patient ;
- et d'autoriser éventuellement une télétransmission des données EGM enregistrées par la prothèse, sans intervention d'un praticien ou auxiliaire médical auprès du patient.

Divers algorithmes de reconstruction de l'ECG de surface à partir des signaux EGM ont ainsi été proposés.

Certaines techniques mettent en oeuvre une combinaison linéaire ou non-linéaire d'une pluralité de signaux EGM.

Ainsi, le US 5 740 811 A1 (Hedberg et al.) prévoit de synthétiser un signal ECG de surface par combinaison d'une pluralité de signaux EGM au moyen d'un réseau de neurones et/ou d'une logique floue et/ou d'un circuit sommateur, après apprentissage effectué par un algorithme du type *feedforward*.

Le US 6 980 850 A1 (Kroll et al.), quant à lui, met en oeuvre un filtrage linéaire avec transformation matricielle. La reconstruction est opérée au moyen d'une matrice de transfert prédéterminée permettant de transformer un vecteur d'entrée représentatif d'une pluralité de signaux EGM en un vecteur résultant représentatif des différentes dérivations ECG. La matrice de transfert est apprise au cours d'une phase d'apprentissage par moyennage de plusieurs matrices instantanées à partir de vecteurs ECG et EGM enregistrés simultanément sur une même durée.

Une autre technique, exposée notamment par le US 7 383 080 (Kil et al.) ou encore par les EP 1 897 587 A2 et US 2008/0065161 (Lian et al.), consiste à produire un signal ressemblant à un ECG de surface ("ECG-like signal" ou "lead-free ECG") par concaténation du signal ventriculaire lointain observé sur l'électrode placée dans l'oreillette avec le signal auriculaire lointain observé sur l'électrode placée dans le ventricule.

Par convention, ici et dans la suite on désignera par :

- "far-field auriculaire" ou $FF_A$ le signal de dépolarisation d'origine ventriculaire (le "bruit électrique" lointain du ventricule) observé sur l'électrode placée dans l'oreillette pendant ou directement après une onde R, et
- "far-field ventriculaire" ou $FF_V$ le signal de dépolarisation d'origine auriculaire (le "bruit électrique" lointain de l'oreillette) observé sur l'électrode placée dans le ventricule pendant ou directement après une onde P.

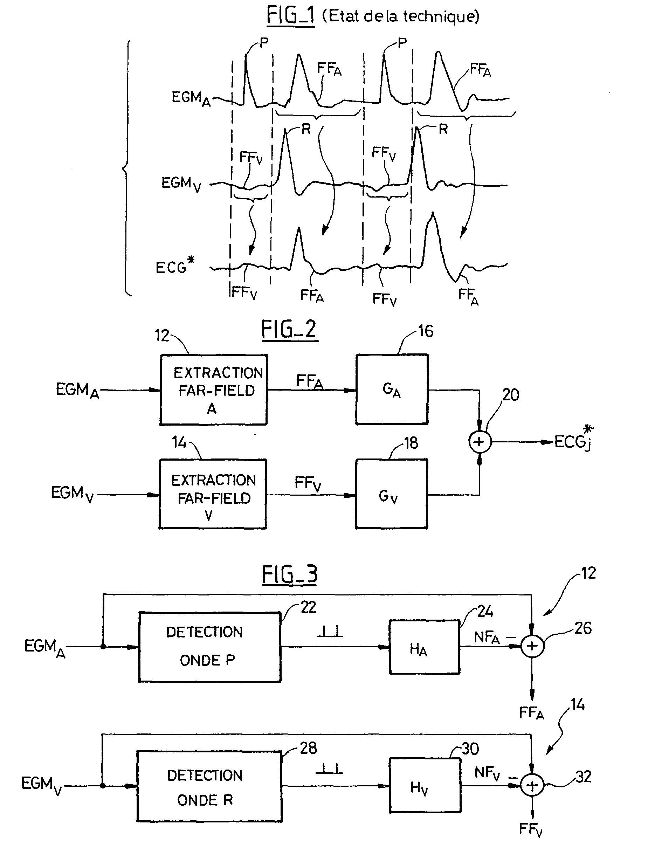

La technique connue évoquée ci-dessus est illustrée sur la Figure 1 annexée.

Sur cette figure, on a représenté le signal de farfield auriculaire $FF_A$ extrait du signal EGM auriculaire $EGM_A$ sur la durée d'une fenêtre temporelle entourant le pic R (activité spontanée ayant son origine dans le ventricule), pic localisé sur le signal EGM ventriculaire $EGM_V$. De la même façon, un signal de farfield ventriculaire $FF_V$ est extrait du signal $EGM_V$ pendant une fenêtre temporelle entourant le pic P (activité spontanée ayant son origine dans l'oreillette), pic localisé sur le signal EGM auriculaire $EGM_A$.

Le signal reconstruit ECG* est obtenu par concaténation des segments $FF_V$ et $FF_A$, après soustraction d'un décalage et multiplication de chacun de ces segments par un facteur donné d'amplification ou d'atténuation, de manière à pouvoir raccorder ces segments entre eux lors de leur concaténation.

Cette technique présente, dans son principe, un certain nombre d'avantages :

- dans le cas d'un patient présentant un rythme régulier, elle est efficace car les deux signaux farfield sont bien séparés ;
- elle est simple à implémenter, et peut donc être mise en oeuvre en temps réel au sein d'un implant ;
- elle ne nécessite pas le recueil préalable d'un ECG, à la différence des méthodes avec combinaison linéaire ou non-linéaire de signaux EGM, qui nécessitent une phase d'apprentissage pour la détermination des coefficients des filtres ou des matrices de transfert ;
- elle permet de détecter des inversions de polarité sur certains signaux ECG, alors que la reconstruction de cette inversion de polarité n'est pas possible si l'on effectue un traitement linéaire ou non-linéaire de la totalité des signaux EGM.

Ces avantages sont toutefois tempérés par un certain nombre d'inconvénients :

- pour un patient présentant un rythme irrégulier, dont potentiellement pathologique, les signaux farfield sont noyés dans les ondes P et R et ne peuvent pas être distingués de façon satisfaisante de ces dernières ;
- le signal "d'électrocardiogramme" reconstruit ne correspond en fait à aucune dérivation ECG réelle. La technique consiste plus à opérer une émulation d'un signal ECG virtuel ("ECG-like signal"), certes utile pour un diagnostic, qu'à véritablement reconstruire un signal ECG qui, idéalement, reproduirait un signal recueilli sur une ou plusieurs des dérivations d'un électrocardiogramme de surface ;
- enfin, cette technique ne permet de produire qu'un seul signal de type ECG, et non une pluralité de dérivations ECG reconstruites, similaires à ce qui serait obtenu lors du recueil d'un électrocardiogramme de surface traditionnel.

Ces divers inconvénients tiennent notamment au fait que les signaux EGM et ECG, s'ils ont la même origine, n'en présentent pas moins des caractéristiques très différentes. En effet, l'activité électrique du coeur est le reflet des stimulations spontanées dues aux courants ioniques dans les cellules cardiaques, ou des stimulations artificielles produites par application d'un courant électrique à ces cellules. Les signaux EGM, recueillis directement par l'implant sur une ou plusieurs voies, sont le reflet des potentiels électriques présents au niveau du myocarde, tandis que les signaux ECG correspondent aux potentiels électriques enregistrés à la surface du corps, sur un certain nombre de dérivations, après propagation depuis le myocarde jusqu'à cette surface.

Un autre inconvénient, propre à toutes les techniques jusqu'à présent proposées, est qu'elles ne permettent pas de vérifier que la reconstruction du signal ECG donne un résultat correct, encore moins de quantifier la qualité de cette reconstruction.

Or il serait souhaitable de pouvoir disposer d'un tel critère, notamment en fonction de l'utilisation que l'on souhaite faire des signaux ECG reconstruits : s'il s'agit de simplement déterminer la présence ou non d'un pic, d'un complexe, ... il est possible de se contenter d'une qualité de reconstruction moyenne, tandis que si l'on souhaite un diagnostic précis, fondé sur des détails spécifiques de l'ECG ou des mesures effectuées sur celui-ci, alors une qualité de reconstruction supérieure sera nécessaire. L'invention a pour but de remédier à ces divers inconvénients, en proposant une nouvelle technique de reconstruction de signaux ECG à partir de signaux EGM qui, tout en conservant les avantages des techniques basées sur une reconstruction à partir des signaux de farfield (notamment la simplicité de mise en oeuvre) élimine les inconvénients précités, notamment une technique :

- qui soit applicable à des situations de rythme irrégulier, où les signaux farfield sont difficiles à distinguer des ondes P et R ;
- qui produise un signal ECG reconstruit le plus proche possible d'un signal ECG réel tel qu'il serait recueilli par un enregistreur de signaux ECG de surface ;
- qui puisse simultanément délivrer une pluralité de signaux ECG reconstruits, correspondant à autant de dérivations ECG différentes.

Le dispositif de l'invention est un dispositif d'un type connu exposé notamment par le EP 1 897 587 A2 précité, correspondant au préambule de la revendication 1. Les éléments propres à l'invention sont énoncés dans la partie caractérisante de cette revendication 1.

Diverses formes de mises en oeuvre avantageuses sont énoncées dans les sous-revendications.

Le dispositif de l'invention peut être implémenté sous diverses formes :

- dispositif implantable du type prothèse cardiaque de stimulation, resynchronisation, cardioversion et/ou défibrillation ;
- programmateur externe comprenant des moyens de téléchargement et d'analyse des signaux EGM recueillis et mémorisés par un implant ;
- moniteur de *home monitoring*, comprenant des moyens de téléchargement et d'analyse des signaux EGM recueillis et mémorisés par un implant, ainsi que des moyens de télétransmission automatique à un site distant des données ainsi recueillies ; et/ou

serveur de données d'un site, recevant des données d'un moniteur distant de *home monitoring* comprenant des moyens de téléchargement des signaux EGM recueillis et mémorisés par un implant, ainsi que des moyens de télétransmission automatique au site des données ainsi recueillies.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1, précitée, expose schématiquement la technique de reconstruction par concaténation de signaux de farfield selon l'état de la technique.

La Figure 2 est une représentation schématique de la technique de reconstruction selon l'invention.

La Figure 3 est une représentation schématique des blocs d'extraction des signaux de farfield ventriculaire et auriculaire de la Figure 1.

La Figure 4 est un schéma exposant la manière dont sont déterminés les coefficients des filtres des blocs de détection des signaux de farfield de la Figure 3.

La Figure 5 est un schéma expliquant la manière dont sont déterminés, par un apprentissage préalable, les coefficients des filtres prédicteurs de signal ECG de la Figure 1.

On va maintenant décrire plusieurs exemples de mise en oeuvre de l'invention. En ce qui concerne ses aspects logiciels, l'invention peut être réalisée par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par ELA Médical, Montrouge, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre de l'invention est à la portée de l'homme du métier, et elle ne sera pas décrite en détail.

L'invention peut toutefois être mise en oeuvre non seulement au sein d'un implant (traitement direct des signaux EGM), mais également dans un programmateur externe utilisé par un praticien par téléchargement et analyse des signaux cardiaques recueillis et mémorisés par un implant. Dans une variante avantageuse, l'invention est également mise en oeuvre dans un moniteur de *home monitoring,* qui est un type particulier de programmateur dont le fonctionnement est entièrement automatisé et ne nécessite pas le recours à un praticien, et qui est notamment destiné à permettre la télétransmission à intervalles réguliers à un site distant des données recueillies et analysées, par exemple quotidiennement, de manière à assurer un suivi à distance du patient.

L'invention peut également être mise en oeuvre au niveau du serveur de données de ce site, les données EGM brutes étant télétransmises à ce serveur directement, sans traitement préalable. Le traitement est alors effectué par le serveur ou par un terminal (PC ou programmateur) connecté à ce dernier.

*Recueil des signaux EGM*

Il s'agit d'acquérir des signaux EGM sur deux "voies", à savoir une voie auriculaire et une voie ventriculaire, chacune de ces voies correspondant à une paire d'électrodes endocavitaires ou épicardiques reliées au boîtier de la prothèse cardiaque implantée.

Le choix des électrodes définissant ces voies dépend de la prothèse cardiaque considérée : stimulateur (pour le traitement des bradycardies), défibrillateur (pour le traitement des tachycardies et fibrillations) ou resynchroniseur (pour le traitement de l'insuffisance cardiaque). On distingue également trois modes de stimulation : simple, double ou triple chambre. À ces différentes fonctions correspondent des électrodes différentes, et des signaux EGM qui ne sont pas les même selon le cas.

Si l'on désigne par "VD", "OD" et "VG", respectivement, les électrodes ventriculaire droite, auriculaire droite et ventriculaire gauche des sondes intracardiaques, avec un signe "+" ou "-" indiquant la position, distale ou proximale, de l'électrode, et par "CoilV" et "SVC" les électrodes de défibrillation respectivement ventriculaire et supraventriculaire, les combinaisons d'électrodes possibles sont les suivantes (où la voie est définie en mode bipolaire uniquement, en considérant l'une des électrodes et le boîtier du générateur) :

- simple chambre : VD+, VD- (et CoilV dans le cas d'un défibrillateur) ;
- double chambre : VD+, VD-, OD+, OD- (et CoilV et SVC dans le cas d'un défibrillateur) ;
- triple chambre : VD+, VD-, OD+, OD-, VG+, VG- (et CoilV et SVC dans le cas d'un défibrillateur).

*Principe de reconstruction de l'ECG*

Les signaux ECG, qui sont la manifestation de l'activité électrique cardiaque à la surface du corps du patient, sont normalement recueillis entre des paires d'électrodes appliquées en des endroits prédéterminés du thorax du patient, chaque paire d'électrodes déterminant une "dérivation". Le tout forme un ensemble de douze dérivations, comprenant des dérivations bipolaires (I, II, III), unipolaires (aVF, aVR, aVL) et précordiales (v1 à v6). L'invention a pour objet de reconstituer les signaux d'une ou plusieurs de ces dérivations ECG à partir de signaux effectivement recueillis sur deux voies EGM, à savoir une voie auriculaire (signal $EGM_A$) et une voie ventriculaire (signal $EGM_V$).

Le principe de base de la technique de reconstruction de l'invention est schématisé sur la Figure 2.

Un signal EGM auriculaire $EGM_A$ et un signal EGM ventriculaire $EGM_V$ sont recueillis de la manière indiquée plus haut sur deux voies EGM respectives, et échantillonnés. Chacun de ces signaux est appliqué à un bloc respectif 12, 14 d'extraction du signal de farfield auriculaire $FF_A$ et d'extraction du signal de farfield ventriculaire $FF_V$. Ces signaux de farfield $FF_A$, $FF_V$ sont appliqués à des filtres respectifs 16, 18 avec des fonctions de transfert $G_A$, $G_V$, linéaires ou non-linéaires. Les sorties des filtres 16, 18 sont additionnées en 20 pour donner un signal $ECG^*_j$ reconstruit, correspondant à l'une des $j$ = 12 dérivations que l'on souhaite reconstruire. Pour obtenir les signaux des autres dérivations ECG que l'on souhaite également (éventuellement) reconstruire, la même technique sera appliquée, avec les mêmes signaux $EGM_A$ et $EGM_V$ en entrée mais avec des paramètres de filtre $G_A$ et $G_V$ différents.

Les filtres $G_A$ et $G_V$ peuvent être des filtres linéaires, ou des filtres non-linéaires tels que des filtres de Volterra ou des réseaux de neurones.

Le filtrage de Volterra est décrit par exemple par Schetzen M, The Volterra and Wiener Theories of Nonlinear Systems, Wiley and Sons, New York, 1980, ou encore par Mathews VJ, Adaptive Polynomial Filters, IEEE Signal Processing Magazine, 8(3), pp.10-26, juillet 1991. Ces filtres permettent en particulier d'établir entre les signaux EGM et les signaux ECG une relation non-linéaire incluant des termes linéaires, quadratiques et cubiques. Ils permettent également d'introduire dans cette relation un retard fini reflétant la propagation des signaux électriques dans les tissus corporels depuis le myocarde jusqu'à la surface de la peau du patient.

En variante, un réseau de neurones approprié peut réaliser les fonctions voulues après un apprentissage en essayant de minimiser au sens des moindres carrés l'erreur quadratique moyenne $\varepsilon^2$. On peut notamment utiliser des réseaux mettant en oeuvre une "approche temps-retard" tels que ceux décrits par Hornik K et Stinchcombe M, Multilayer Feedforward Networks are Universal Approximators, Neural Networks, Vol. 2, No. 5 pp. 359-366, 1989 (pour une approche dite FTDNN, *Focused Time-Delayed Neural Networks*), Elman JL, Finding Structure in Time, Cognitive Science, Vol. 14, pp. 179-211, 1990 (pour une approche dite de type Elman), ou encore Rodriguez P, Wiles J et Elman JL, A Recurrent Neural Network that Learns to Count, Connection Science, Vol 11, no 1, pp. 5-40, 1999 (pour une approche de type RTDNN, *Recurrent Time-Delayed Neural Networks*).

On a illustré plus en détail Figure 3 la structure des blocs 12, 14 d'extraction des signaux de farfield auriculaire et ventriculaire.

La première étape consiste à opérer la détection des ondes P et R dans les signaux EGM auriculaire $EGM_A$ et ventriculaire $EGM_V$, au moyen des blocs de détection 22, 28. Cette opération est effectuée par l'intermédiaire d'algorithmes bien connus, non modifiés. La sortie des blocs de détection 22, 28 se présente sous la forme d'une suite d'impulsions, impulsions dont la position temporelle indique l'instant de survenue du pic P ou R, et dont l'amplitude correspond à l'amplitude du signal EGM.

Les signaux délivrés par les blocs 22, 28 sont ensuite appliqués à des filtres respectifs 24, 30 d'estimation du signal de nearfield auriculaire $NF_A$ et du signal de nearfield ventriculaire $NF_V$.

Si l'on désigne par $D_P$ la fonction de transfert du bloc 22 de détection de l'onde P à partir du signal $EGM_A$, par $D_R$ la fonction de transfert du bloc 28 de détection de l'onde R à partir du signal $EGM_V$, par $H_A$ la fonction de transfert du filtre 24 d'estimation du signal de nearfield auriculaire $NF_A$ et par $H_V$ la fonction de transfert du filtre 30 d'estimation du signal de nearfield ventriculaire $NF_V$, il vient :

$$NF_A \ = \ H_A \, (D_P(EGM_A)),$$

et

$$NF_V \ = \ H_V \, (D_R(EGM_V))$$

Les filtres $H_A$ et $H_V$ sont linéaires et doivent couvrir une durée égale à la largeur d'un complexe QRS, ou son équivalent visible sur les signaux EGM. Dans le cas de signaux EGM échantillonnés à 128 Hz, la taille des filtres $H_A$ et $H_V$ sera de 20 à 30 coefficients, ce qui permet d'obtenir en sortie une réponse impulsionnelle très proche des signaux EGM autour des pics P et R.

Le signal de farfield auriculaire $FF_A$ est obtenu par soustraction, en 26, du signal de nearfield auriculaire $NF_A$ au signal auriculaire d'origine $EGM_A$. Le signal de farfield ventriculaire est obtenu de la même façon par soustraction en 32 du signal de nearfield ventriculaire $NF_V$ au signal ventriculaire d'origine $EGM_V$, soit :

$$FF_A = EGM_A - NF_A,$$

et

$$FF_V = EGM_V - NF_V.$$

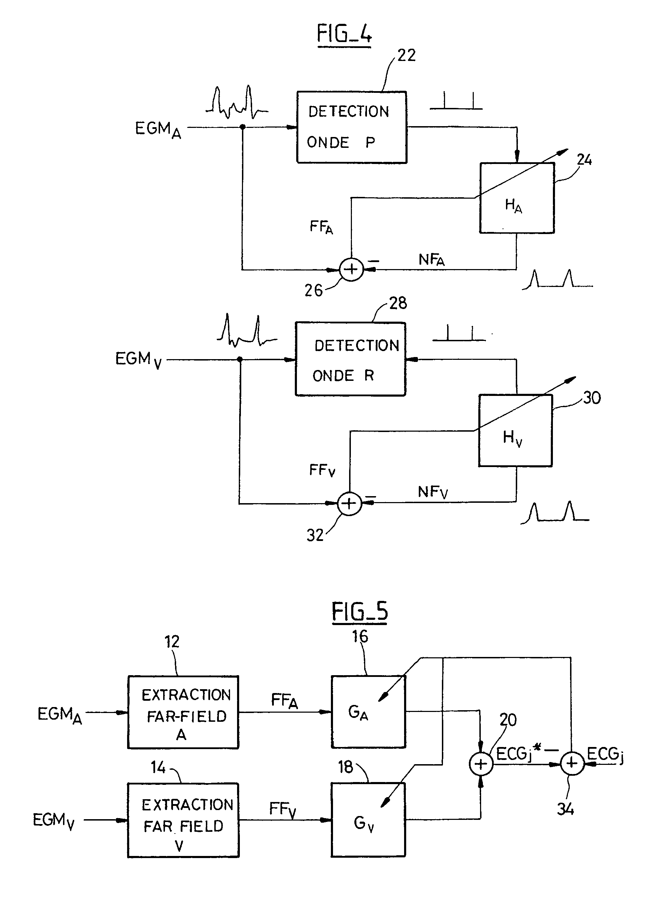

Les coefficients des filtres $H_A$ et $H_V$ (blocs 24 et 30 sur la Figure 3) sont obtenus par une technique d'apprentissage de la forme des ondes P et R, illustrée Figure 4.

Cet apprentissage met en oeuvre un filtrage adaptatif, actif seulement pendant les intervalles de temps contenant des pics R et P bien séparés. La longueur des filtres doit couvrir la durée d'un complexe QRS, et l'apprentissage se fait sur plusieurs cycles cardiaques, en comparant en 26 le signal $NF_A$ de nearfield auriculaire, résultant de l'estimation par la chaîne de blocs 22, 24, avec le signal originel $EGM_A$, et, de la même façon, en comparant en 32 le signal $NF_V$ de nearfield ventriculaire, résultant de l'estimation par la chaîne de blocs 28, 30, avec le signal originel $EGM_V$.

Le signal d'erreur respectif, constitué par le signal de farfield $FF_A$ (ou $FF_V$), commande par une technique connue l'adaptation du filtre $H_A$ (ou $H_V$), par exemple grâce à un algorithme d'adaptation de type moindre carrés moyens LMS ou, avantageusement, par un algorithme mettant en oeuvre la méthode des moindres carrés récursive RLS. Cette dernière méthode est décrite par exemple par Hayes, MH (1996), Recursive Least Squares, Statistical Digital Signal Processing and Modeling, Wiley, p. 541 (ISBN 0-471-59431-8), ou encore par Haykin S, Adaptive Filter Theory, Prentice Hall, 2002 (ISBN 0-13-048434-2). Elle permet de résoudre en temps réel un système linéaire et demande mois de ressources informatiques pour le calcul des coefficients, de sorte qu'elle peut être mise en oeuvre directement dans un implant sans le besoin d'un programmateur externe. De plus, le fait d'utiliser un pas variable pour contrôler la convergence lors des itérations représente un avantage par rapport aux méthodes itératives à pas fixe comme le LMS.

On notera que l'apprentissage des filtres $H_A$ et $H_V$ ne nécessite pas de recueil d'un signal ECG. Cet apprentissage peut donc fonctionner en boucle fermée, en permanence, ou en boucle ouverte après un apprentissage périodique ou consécutif à un événement prédéterminé.

On va maintenant décrire, en référence à la Figure 5, la manière dont sont déterminés les coefficients des filtres de reconstruction $G_A$ et $G_V$ (blocs 16 et 18 de la Figure 2).

Ces filtres, qui permettent d'obtenir un signal ECG reconstruit $ECG^*_j$ à partir des signaux de farfield auriculaire $FF_A$ et ventriculaire $FF_V$, sont déterminés par apprentissage de la forme des signaux ECG. Cet apprentissage est opéré, dans une première phase, par recueil simultané d'un jeu de données de référence constitué de signaux d'électrogramme auriculaire $EGM_A$ et ventriculaire $EGM_V$, et de signaux d'électrocardiogramme $ECG_j$ synchronisés avec ces signaux EGM.

Les filtres $G_A$ et $G_V$ sont, comme indiqué plus haut, des filtres linéaires ou non-linéaires (filtres de Volterra ou réseaux de neurones, notamment) et il convient de calculer les deux filtres $G_A$ et $G_V$ correspondant à chaque dérivation $j$. Les coefficients de ces filtres dépendent : (i) du patient, (ii) des signaux EGM utilisés en entrée pour la reconstruction, et (iii) de la dérivation ECG que l'on souhaite reconstruire.

Pour ce faire, un ECG reconstruit $ECG^*_j$ est estimé à partir des signaux $EGM_A$ et $EGM_V$ par la technique exposé plus haut en référence à la Figure 2 (extraction des signaux de farfield par des blocs 12, 14, et estimation de l'ECG par des blocs 16, 18, respectifs).

La différence entre l'ECG réel $ECG_j$ et l'ECG reconstruit $ECG^*_j$ est évalué en 34, le signal d'écart permettant de piloter les filtres adaptatifs $G_A$ et $G_V$ selon des schémas d'adaptation connus tels que le LMS, le RLS (décrits plus haut en référence à l'adaptation des filtres $H_A$ et $H_V$ sur la Figure 4), ou autre technique d'apprentissage connue.

Une fois déterminés les coefficients des filtres $G_A$ et $G_V$, ces filtres sont figés (fonctionnement en boucle ouverte) pour pouvoir ensuite reconstruire, en temps réel, une dérivation $ECG^*_j$, de la manière exposée en référence à la Figure 2.

*Évaluation de la qualité de la reconstruction de l'ECG*

Un autre aspect de la présente invention réside dans l'évaluation de la qualité de la reconstruction de l'ECG.

Il est en effet intéressant de pouvoir estimer la qualité de cette reconstruction, par exemple pour choisir telle ou telle technique de reconstruction sur la base d'un compromis acceptable entre les contraintes matérielles (temps de calcul, ressources matérielles et logicielles disponibles, ...) et les besoins réels selon l'utilisation que l'on souhaite faire de l'ECG reconstruit (détection de la simple présence de certaines caractéristiques, ou au contraire examen plus poussé des formes d'onde).

Pour évaluer cette qualité, les signaux EGM et ECG (signaux ECG réellement recueillis) sont acquis simultanément, pendant une durée $T_m$ de mesure. Cette durée $T_m$ doit représenter au moins deux cycles cardiaques (2 s environ) et peut aller jusqu'à 100 ou 1000 s. Les séquences choisies comme jeu de données de référence pour l'apprentissage ont une durée $T_r$ d'une seconde au moins et peuvent aller jusqu'à 99 ou 999 s. Les signaux EGM et ECG sont acquis simultanément pendant une durée $T_m$, typiquement avec un rythme d'échantillonnage de 128 Hz.

Si les fréquences d'échantillonnage des ECG et des EGM (en général situées dans la plage 100 Hz à 1 kHz) sont différentes, il faudra synchroniser les données par une technique appropriée, telle qu'une interpolation (linéaire, polynomiale ou par des *splines*) ou une compression (par exemple par l'algorithme de *turning point* de Mueller).

La qualité de la reconstruction des signaux ECG sera évaluée par un critère numérique consistant à déterminer, sur une séquence qui n'a pas été utilisée pour l'apprentissage, le coefficient de corrélation $\rho$ entre les signaux ECG réels $y[k]$ et les signaux ECG reconstruits $y_{rec}[k]$.

Concrètement, une erreur de séquencement de l'ordre de 40 ms (soit un décalage de $d = 5$ échantillons pour une fréquence d'échantillonnage de 128 Hz) dans les signaux reconstruits n'altère pas les capacités de diagnostic des signaux ECG.

Le coefficient de corrélation est évalué pour chaque décalage $k$ :

$$\rho_k = \frac{\sum_{i=0}^{J}(y[i]-\bar{y})(y_{rec}[i+k]-\bar{y}_{rec})}{\sqrt{\sum_{i=0}^{J}(y[i]-\bar{y})^2}\sqrt{\sum_{i=0}^{J}(y_{rec}[i+k]-\bar{y}_{rec})^2}}$$

La qualité de reconstruction (comprise entre -1 et +1) est estimée par :

$$\rho = \max_{-d \le k \le d}(\rho_k)$$

Sur des séquences ECG qui présentent un rythme régulier, le procédé de l'invention assure une qualité de reconstruction supérieure à 80 % à partir de signaux unipolaires provenant de l'oreillette et du ventricule (le signal proximal étant considéré par rapport au boîtier) pour un patient présentant un rythme régulier. Les signaux ECG reconstruit reproduisent fidèlement la polarité, la largeur et la position des complexes QRS dans les ECG. Chez certains patients présentant un rythme irrégulier, le procédé de l'invention assure une qualité de reconstruction de l'ordre de 70 % à 75 % sur certaines séquences ECG, alors que les autres méthodes basées sur un traitement direct de l'EGM sans extraction du farfield ne donnent qu'une qualité de reconstruction inférieure à 50 %.

Concrètement, la qualité de reconstruction doit être de l'ordre d'au moins 60 à 65 % pour pouvoir retrouver dans l'ECG reconstruit certaines singularités dont souhaite déterminer la présence ou l'absence (pics, complexes, ...), ce qui est suffisant pour un simple suivi de type monitoring ECG. En revanche, pour pouvoir établir un diagnostic plus précis à partir de l'examen détaillé des formes d'ondes, la qualité de reconstruction doit être d'au moins 80 %.

Le critère de qualité de reconstruction peut en particulier être utilisé pour valider le calcul des coefficients des filtres lors de la procédure d'apprentissage.

Ainsi, à l'issue du calcul des paramètres des filtres, la qualité de reconstruction est comparée à un seuil donné. Ce seuil est programmable et peut être éventuellement modifié par le praticien, ou peut être prédéfini à une valeur acceptable, par exemple 60 %.

Si le critère de qualité est vérifié (seuil dépassé), les coefficients calculés sont sauvegardés, et les filtres calculés seront utilisés pour la reconstruction ultérieure de la dérivation ECG considérée. Le processus est éventuellement répété pour une autre dérivation ECG.

En revanche, si le critère n'est pas vérifié, il y a lieu de recommencer la détermination des paramètres des filtres, soit en sélectionnant une autre période de référence $T_r$ dans la fenêtre de mesure $T_m$ (dans la fenêtre $T_r$ il y avait peut-être des arythmies susceptibles de perturber l'apprentissage), ou bien en recommençant l'acquisition d'un autre jeu de données sur une autre durée $T_m$.

## Revendications

1. Un dispositif de traitement de signaux EGM d'électrogramme endocavitaire ou épicardique représentatifs de potentiels cardiaques de dépolarisation du myocarde et recueillis sur des voies EGM d'un dispositif médical implantable actif, ce dispositif de traitement comportant :

   - des moyens d'acquisition d'un signal EGM ventriculaire ($EGM_V$) et d'un signal EGM auriculaire ($EGM_A$) ;
   - des moyens d'extraction d'une composante de farfield ventriculaire ($FF_V$) à partir du signal EGM ventriculaire acquis ($EGM_V$) et d'extraction d'une composante de farfield auriculaire ($FF_A$) à partir du signal EGM auriculaire acquis ($EGM_A$) ; et
   - des moyens de reconstruction d'ECG, aptes à combiner les signaux de farfield ventriculaire et de farfield auriculaire pour délivrer en sortie un signal ECG d'électrogramme de surface reconstruit ($ECG^*_j$) ; et
   - les moyens de reconstruction d'ECG comprennent :

      · un filtre estimateur de farfield ventriculaire (18), recevant en entrée la composante de farfield ventriculaire et délivrant en sortie un signal de farfield ventriculaire filtré ;

· un filtre estimateur de farfield auriculaire (16), recevant en entrée la composante de farfield auriculaire et délivrant en sortie un signal de farfield auriculaire filtré ; et
· des moyens sommateurs (20), aptes à additionner les signaux de farfield filtrés pour délivrer en sortie ledit signal ECG reconstruit (ECG$_j$*); et

**caractérisé en ce que** :

- les moyens d'extraction des composantes de farfield ventriculaire et de farfield auriculaire comprennent, pour chacune des voies ventriculaire ou auriculaire :

· des moyens (22 ; 28) de détection d'une onde de dépolarisation spontanée ou stimulée (P ; R), recevant en entrée le signal EGM et délivrant en sortie un signal impulsionnel correspondant ;
· un filtre estimateur de nearfield (24 ; 30), recevant en entrée le signal impulsionnel et délivrant en sortie une composante de nearfield (NF$_A$ ; NF$_V$) ; et
· des moyens soustracteurs (26 ; 32), aptes à soustraire du signal EGM (EGM$_A$ ; EGM$_V$) la composante de nearfield (NFA ; NF$_V$) pour délivrer en sortie ladite composante de farfield (FF$_A$ ; FF$_V$).

2. Le dispositif de la revendication 1, comportant en outre des moyens de détermination des paramètres (H$_A$ ; H$_V$) des filtres estimateurs de nearfield (24 ; 30), comprenant des moyens de calcul itératif aptes à mettre en oeuvre un algorithme de filtrage adaptatif.

3. Le dispositif de la revendication 1, comportant en outre des moyens de détermination préalable des paramètres (G$_A$, G$_V$) des filtres estimateurs de farfield (16, 18), comprenant :

- des moyens d'acquisition simultanée du signal EGM ventriculaire (EGM$_V$), du signal EGM auriculaire (EGM$_A$), et d'un signal ECG recueilli (ECG$_j$); et
- des moyens de détermination desdits paramètres (G$_A$, G$_V$) par minimisation d'un écart ($\varepsilon$) entre le signal ECG recueilli (ECG$_j$) et un signal ECG reconstruit (ECG$_j$*) au moyen des filtres estimateurs (16, 18) de farfield à partir des signaux EGM ventriculaire et auriculaire (EGM$_A$ ; EGM$_V$).

4. Le dispositif de la revendication 3, dans lequel les moyens de détermination des paramètres (G$_A$, G$_V$) des filtres estimateurs de farfield (16, 18) comprennent des moyens de calcul itératif aptes à mettre en oeuvre un algorithme de filtrage adaptatif.

5. Le dispositif de la revendication 1, comportant en outre des moyens d'évaluation de la qualité de la reconstruction opérée par les moyens de reconstruction d'ECG, comprenant :

- des moyens d'acquisition simultanée du signal EGM ventriculaire (EGM$_V$), du signal EGM auriculaire (EGM$_A$), et d'un signal ECG recueilli (ECG$_j$) ; et
- des moyens de calcul d'un coefficient de corrélation entre, d'une part, le signal ECG recueilli (ECG$_j$) et, d'autre part, un signal ECG reconstruit (ECG$_j$*) au moyen des filtres estimateurs de farfield ventriculaire et auriculaire à partir des signaux EGM ventriculaire et auriculaire.

6. Le dispositif de la revendication 5, comprenant en outre :

- des moyens de détermination préalable des paramètres (G$_A$, G$_V$) des filtres estimateurs de farfield (16, 18) ventriculaire et auriculaire, et
- des moyens de validation des paramètres ainsi déterminés, ces moyens comprenant des moyens pour comparer à un seuil donné le coefficient de corrélation calculé par lesdits moyens d'évaluation de la qualité de la reconstruction, et pour valider ou infirmer lesdits paramètres en fonction du résultat de cette comparaison.

7. Le dispositif de la revendication 1, dans lequel le dispositif est apte à recevoir les signaux EGM ventriculaire et auriculaire (EGM$_A$, EGM$_V$) acquis à partir d'électrodes choisies parmi : électrode ventriculaire droite distale et/ou proximale, électrode auriculaire droite distale et/ou proximale, électrode ventriculaire gauche distale et/ou proximale, bobine de défibrillation ventriculaire ou auriculaire, bobine de défibrillation supra-ventriculaire.

8. Le dispositif de la revendication 1, où ledit dispositif est un dispositif implantable du type prothèse cardiaque de stimulation, resynchronisation, cardioversion et/ou défibrillation.

9. Le dispositif de la revendication 1, où ledit dispositif est un programmateur externe comprenant des moyens de téléchargement et d'analyse des signaux EGM recueillis et mémorisés par un implant.

10. Le dispositif de la revendication 1, où ledit dispositif est un moniteur de *home monitoring,* comprenant des moyens de téléchargement et d'analyse des signaux EGM recueillis et mémorisés par un implant, ainsi que des moyens de télétransmission automatique à un site distant des données ainsi recueillies.

11. Le dispositif de la revendication 1, où ledit dispositif est un serveur de données d'un site, recevant des données d'un moniteur distant de *home monitoring* comprenant des moyens de téléchargement des signaux EGM recueillis et mémorisés par un implant, ainsi que des moyens de télétransmission automatique audit site des données ainsi recueillies.

**Claims**

1. Device for processing endocavitary or epicardiac electrogram EGM signals representative of myocardial depolarization cardiac potentials and collected over EGM channels from an active implantable medical device, this processing device comprising:

   - means for acquiring a ventricular EGM signal ($EGM_V$) and an auricular EGM signal ($EGM_A$);
   - means for extracting a ventricular far field component ($FF_V$) from the ventricular EGM signal acquired ($EGM_V$) and extracting an auricular far field component ($FF_A$) from the auricular EGM signal acquired ($EGM_A$); and
   - ECG reconstruction means, capable of combining the ventricular far field and auricular far field signals to deliver as output a reconstructed surface electrogram ECG signal ($ECG_j^*$);
   - the ECG reconstruction means comprise:

     . a ventricular far field estimating filter (18), receiving as input the ventricular far field component and delivering as output a filtered ventricular far field signal;
     . an auricular far field estimating filter (16), receiving as input the auricular far field component and delivering as output a filtered auricular far field signal; and
     . summing means (20), capable of adding the filtered far field signals to deliver as output said reconstructed ECG signal ($ECG_j^*$) ; and **characterized in that**:

   - the means for extracting the ventricular far field and auricular far field components comprise, for each of the ventricular or auricular channels:

     . means (22; 28) for detecting a spontaneous or stimulated depolarization wave (P; R), receiving as input the EGM signal and delivering as output a corresponding pulsed signal;
     . a near field estimating filter (24; 30), receiving as input the pulsed signal and delivering as output a near field component ($NF_A$; $NF_V$) ; and
     . subtracting means (26; 32), capable of subtracting from the EGM signal ($EGM_A$; $EGM_V$) the near field component ($NF_A$; $NF_V$) to deliver as output said far field component ($FF_A$; $FF_V$).

2. Device according to Claim 1, also comprising means for determining parameters ($H_A$; $H_V$) of the near field estimating filters (24; 30), comprising iterative computation means capable of implementing an adaptive filtering algorithm.

3. Device according to Claim 1, also comprising means for first determining the parameters ($G_A$, $G_V$) of the far field estimating filters (16, 18), comprising:

   - means for simultaneously acquiring the ventricular EGM signal ($EGM_V$), the auricular EGM signal ($EGM_A$), and a collected ECG signal ($ECG_j$) ; and
   - means for determining said parameters ($G_A$, $G_V$) by minimizing a difference ($\varepsilon$) between the collected ECG signal ($ECG_j$) and a reconstructed ECG signal ($ECG_j^*$) by means of the far field estimating filters (16, 18) from the ventricular and auricular EGM signals ($EGM_A$; $EGM_V$),

4. Device according to Claim 3, in which the means for determining the parameters ($G_A$, $G_V$) of the far field estimating filters (16, 18) comprise iterative computation means capable of implementing an adaptive filtering algorithm.

5. Device according to Claim 1, also comprising means for assessing the quality of the reconstruction provided by the ECG reconstruction means, comprising:

- means for simultaneously acquiring the ventricular EGM signal ($EGM_V$), the auricular EGM signal ($EGM_A$), and a collected ECG signal ($ECG_j$); and
- means for computing a correlation coefficient between, on the one hand, the collected ECG signal ($ECG_j$) and, on the other hand, a reconstructed ECG signal ($ECG_j^*$) by means of the ventricular and auricular far field estimating filters from the ventricular and auricular EGM signals.

6. Device according to Claim 5, also comprising:

- means for first determining the parameters ($G_A$, $G_V$) of the ventricular and auricular far field estimating filters (16, 18), and
- means for validating the duly determined parameters, these means comprising means for comparing with a given threshold the correlation coefficient computed by said means for assessing the quality of the reconstruction, and for validating or invalidating said parameters according to the result of this comparison.

7. Device according to Claim 1, in which the device is capable of receiving the ventricular and auricular EGM signals ($EGM_A$, $EGM_V$) acquired from electrodes selected from: distal and/or proximal right ventricular electrode, distal and/or proximal right auricular electrode, distal and/or proximal left ventricular electrode, ventricular or auricular defibrillation coil, supra-ventricular defibrillation coil.

8. Device according to Claim 1, in which said device is an implantable device of the stimulation, resynchronization, cardioversion and/or defibrillation cardiac prosthesis type.

9. Device according to Claim 1, in which said device is an external programmer comprising means for downloading and analysing the EGM signals collected and stored by an implant.

10. Device according to Claim 1, in which said device is a *home monitoring* monitor, comprising means for downloading and analysing the EGM signals collected and stored by an implant, and means for automatically uploading the duly collected data to a remote site.

11. Device according to Claim 1, in which said device is a data server of a site, receiving data from a remote *home monitoring* monitor comprising means for downloading the EGM signals collected and stored by an implant, and means for automatically uploading the duly collected data to said site.

**Patentansprüche**

1. Vorrichtung zur Verarbeitung von EGM-Signalen eines endokavitären oder epikardischen Elektrogramms, die Depolarisations-Herzpotentiale des Myokards repräsentieren und auf EGM-Wegen einer aktiven implantierbaren medizinischen Vorrichtung gesammelt werden, wobei diese Verarbeitungsvorrichtung umfasst:

- Mittel zum Erfassen eines ventrikulären EGM-Signals ($EGM_V$) und eines artrialen EGM-Signals ($EGM_A$);
- Mittel zum Extrahieren einer ventrikulären Fernfeldkomponente ($FF_V$) aus dem erfassten ventrikulären EGM-Signal ($EGM_V$) und zum Extrahieren einer artrialen Fernfeldkomponente ($FF_A$) aus dem erfassten artrialen EGM-Signal ($EGM_A$); und
- Mittel zum Rekonstruieren eines ECG, die die ventrikulären und artrialen Fernfeldsignale kombinieren können, um am Ausgang ein ECG-Signal eines rekonstruierten Oberflächenelektrogramms ($ECG_j^*$) auszugeben; und
- wobei die ECG-Rekonstruktionsmittel umfassen:

. ein Schätzfilter (18) für das ventrikuläre Fernfeld, das am Eingang die ventrikuläre Fernfeldkomponente empfängt und am Ausgang ein gefiltertes ventrikuläres Fernfeldsignal ausgibt;
. ein Schätzfilter (16) für das artriale Fernfeld, das am Eingang die artriale Fernfeldkomponente empfängt und am Ausgang ein gefiltertes artriales Fernfeldsignal ausgibt; und
. Summationsmittel (20), die die gefilterten Fernfeldsignale addieren können, um am Ausgang das rekonstruierte ECG-Signal ($ECG_j^*$) auszugeben; und
**dadurch gekennzeichnet, dass**:

- die Mittel zum Extrahieren der ventrikulären und artrialen Fernfeldkomponenten für jeden der ventrikulären oder artrialen Wege umfassen:

. Mittel (22; 28) zum Detektieren einer spontanen oder stimulierten Depolarisationswelle (P; R), die am Eingang das EGM-Signal empfangen und am Ausgang ein entsprechendes Impulssignal ausgeben;
. ein Nahfeld-Schätzfilter (24; 30), das am Eingang das Impulssignal empfängt und am Ausgang eine Nahfeldkomponente ($NF_A$; $NF_V$) ausgibt; und
. Subtraktionsmittel (26; 32), die von dem EGM-Signal ($EGM_A$; $EGM_V$) die Nahfeldkomponente ($NF_A$, $NF_V$) subtrahieren können, um am Ausgang die Fernfeldkomponente ($FF_A$; $FF_V$) auszugeben.

2. Vorrichtung nach Anspruch 1, die außerdem Mittel zum Bestimmen von Parametern ($H_A$; $H_V$) der Nahfeld-Schätzfilter (24; 30) umfasst, die Mittel zum iterativen Berechnen enthalten, die einen Algorithmus zur adaptiven Filterung ausführen können.

3. Vorrichtung nach Anspruch 1, die außerdem Mittel zum vorherigen Bestimmen von Parametern ($G_A$, $G_V$) der Fernfeld-Schätzfilter (16, 18) enthält, die umfassen:

- Mittel zum gleichzeitigen Erfassen des ventrikulären EGM-Signals ($EGM_V$), des artrialen EGM-Signals ($EGM_A$) und eines gesammelten ECG-Signals ($ECG_j$) ; und
- Mittel zum Bestimmen der Parameter ($G_A$, $G_V$) durch Minimieren eines Abstands ($\varepsilon$) zwischen dem gesammelten ECG-Signal ($ECG_j$) und einem rekonstruierten ECG-Signal ($ECG_j^*$) mittels der Fernfeld-Schätzfilter (16, 18) anhand der ventrikulären und artrialen EGM-Signale ($EGM_A$; $EGM_V$),

4. Vorrichtung nach Anspruch 3, wobei die Mittel zum Bestimmen der Parameter ($G_A$, $G_V$) der Fernfeld-Schätzfilter (16, 18) iterative Rechenmittel umfassen, die einen Algorithmus zur adaptiven Filterung ausführen können.

5. Vorrichtung nach Anspruch 1, die außerdem Mittel zum Bewerten der Qualität der Rekonstruktion umfasst, die durch die ECG-Rekonstruktionsmittel betrieben werden, mit:

- Mitteln zum gleichzeitigen Erfassen des ventrikulären EGM-Signals ($EGM_V$), des artrialen EGM-Signals ($EGM_A$) und eines gesammelten ECG-Signals ($ECG_j$); und
- Mitteln zum Berechnen eines Korrelationskoeffizienten einerseits zwischen dem gesammelten ECG-Signal ($ECG_j$) und andererseits einem rekonstruierten ECG-Signal ($ECG_j^*$) mittels der ventrikulären und artrialen Fernfeld-Schätzfilter anhand der ventrikulären und artrialen EGM-Signale.

6. Vorrichtung nach Anspruch 5, die außerdem umfasst:

- Mittel zum vorherigen Bestimmen der Parameter ($G_A$, $G_V$) der ventrikulären und artrialen Fernfeld-Schätzfilter (16, 18) und
- Mittel zum Validieren der somit bestimmten Parameter, wobei diese Mittel Mittel umfassen, um den Korrelationskoeffizienten, der durch die Mittel zur Bewertung der Qualität der Rekonstruktion berechnet wird, mit einem gegebenen Schwellenwert zu vergleichen und um die Parameter als Funktion des Ergebnisses dieses Vergleichs für gültig oder ungültig zu erklären.

7. Vorrichtung nach Anspruch 1, wobei die Vorrichtung die ventrikulären und artrialen EGM-Signale ($EGM_A$, $EGM_V$) empfangen kann, die anhand von Elektroden erfasst werden, die gewählt sind aus distaler und/oder proximaler rechter ventrikulärer Elektrode, distaler und/oder proximaler rechter artrialer Elektrode, distaler und/oder proximaler linker ventrikulärer Elektrode, ventrikulärer oder artrialer Defibrillationsspule, supraventrikulärer Defibrillationsspule.

8. Vorrichtung nach Anspruch 1, wobei die Vorrichtung eine implantierbare Vorrichtung des Typs Stimulations-, Resynchronisations-, Kardioversions- und/oder Defibrillations-Herzprothese ist.

9. Vorrichtung nach Anspruch 1, wobei die Vorrichtung eine externe Programmiereinrichtung ist, die Mittel zum Fernladen und Analysieren der durch ein Implantat gesammelten und gespeicherten EGM-Signale umfasst.

10. Vorrichtung nach Anspruch 1, wobei die Vorrichtung ein *Home Monitoring*-Monitor ist, der Mittel zum Fernladen und Analysieren der durch ein Implantat gesammelten und gespeicherten EGM-Signale sowie Mittel zum automatischen Fernübertragen zu einem entfernten Ort der auf diese Weise gesammelten Daten umfasst.

11. Vorrichtung nach Anspruch 1, wobei die Vorrichtung ein Server für Daten eines Ortes ist, der Daten eines entfernten *Home Monitoring*-Monitors empfängt und Mittel zum Fernladen der durch ein Implantat gesammelten und gespeicherten EGM-Signale sowie Mittel zur automatischen Fernübertragung an den Ort der auf diese Weise gesammelten Daten umfasst.

FIG_1 (Etat de la technique)

FIG_2

FIG_3

EP 2 238 896 B1

## FIG_4

## FIG_5

15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5740811 A1, Hedberg
- US 6980850 A1, Kroll
- US 7383080 B, Kil
- EP 1897587 A2
- US 20080065161 A, Lian

**Littérature non-brevet citée dans la description**

- **Schetzen M.** The Volterra and Wiener Theories of Nonlinear Systems. Wiley and Sons, 1980
- **Mathews VJ.** Adaptive Polynomial Filters. *IEEE Signal Processing Magazine,* Juillet 1991, vol. 8 (3), 10-26
- **Hornik K ; Stinchcombe M.** Multilayer Feedforward Networks are Universal Approximators. *Neural Networks,* 1989, vol. 2 (5), 359-366
- **Elman JL.** Finding Structure in Time. *Cognitive Science,* 1990, vol. 14, 179-211
- **Rodriguez P ; Wiles J ; Elman JL.** A Recurrent Neural Network that Learns to Count. *Connection Science,* 1999, vol. 11 (1), 5-40
- Recursive Least Squares. **Hayes, MH.** Statistical Digital Signal Processing and Modeling. Wiley, 1996, 541
- **Haykin S.** Adaptive Filter Theory. Prentice Hall, 2002